(19) Europäisches Patentamt
European Patent Office
Office européen des brevets

(11) Veröffentlichungsnummer: **0 322 396**
**A1**

# (12) EUROPÄISCHE PATENTANMELDUNG

(21) Anmeldenummer: **88890278.0**

(22) Anmeldetag: **09.11.88**

(51) Int. Cl.4: **C 07 D 401/04**
A 61 K 31/415, A 61 K 31/445

(30) Priorität: **15.12.87 AT 3306/87**

(43) Veröffentlichungstag der Anmeldung:
**28.06.89 Patentblatt 89/26**

(84) Benannte Vertragsstaaten:
**BE CH DE ES FR GB GR IT LI LU NL SE**

(71) Anmelder: **Gerot-Pharmazeutika Gesellschaft m.b.H.
Arnethgasse 3
A-1171 Wien (AT)**

(72) Erfinder: **Schlager, Ludwig H., Dr.
Nottebohmstrasse 12
A-1190 Wien (AT)**

(74) Vertreter: **Pawloy, Heinrich, Dr. et al
Riemergasse 14
A-1010 Wien (AT)**

## (54) Neue Derivate von Benzimidazol, ihre Herstellung und Verwendung.

(57) Es werden neue Derivate von 4-(2-Oxo-1-benzimidazolinyl)-piperidin der allgemeinen Formel

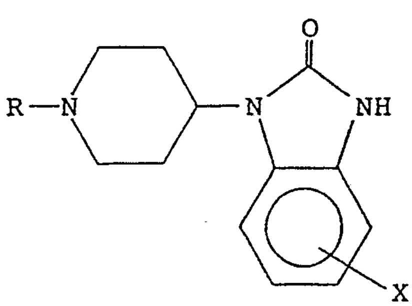

(I),

worin X Wasserstoff oder Halogen und R entweder einen aliphatischen Hydroxyacylrest oder einen Dihydropyridin-3-carbonyloxyalkyl- bzw. einen Dihydropyridin-3-carbonyloxyacyl-Rest bedeuten, sowie ein Verfahren zu deren Herstellung geoffenbart. Die neuen Derivate besitzen blutdrucksenkende Wirksamkeit.



Bundesdruckerei Berlin

## Beschreibung

### Neue Derivate von Benzimidazol, ihre Herstellung und Verwendung

Die Erfindung betrifft neue Derivate von 4-(2-Oxo-1-benzimidazolinyl)-piperidin der allgemeinen Formel

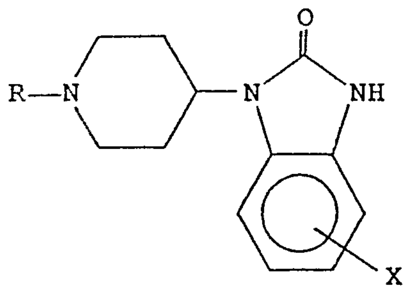

(I),

worin X Wasserstoff oder Halogen und R entweder einen aliphatischen Hydroxyacylrest oder einen Dihydropyridin-3-carbonyloxyalkyl- bzw. einen Dihydropyridin-3-carbonyloxyacyl-Rest der allgemeinen Struktur

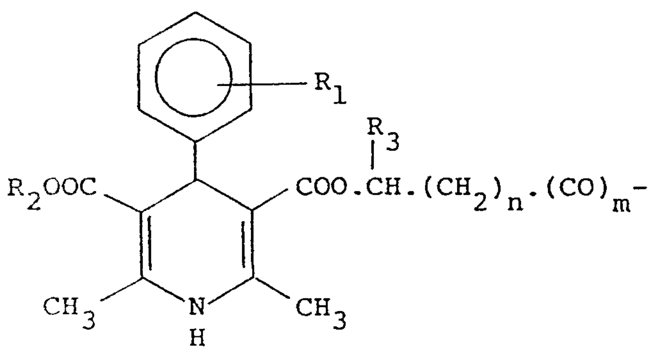

(II)

bedeutet, worin $R_1$ Wasserstoff, Halogen, Nitro, Cyanmethoxy- oder N-subst. Carbamylmethoxy-, $R_2$ ein niederer Alkylrest mit 1 bis 6 C-Atomen, $R_3$ Wasserstoff oder ein niederer Alkylrest, n die Zahl 1 bis 5 und m die Zahl Null oder 1 sein kann.

Der Grundstoff der algemeinen Formel (I) mit X und R = Wasserstoff ist seit 1960 bekannt (Helv.Chim.Acta 43, 1306, 1312 (1960)). Seither wurden zahlreiche Derivate der allgemeinen Formel (I) mit anderen Resten R als den oben bezeichneten hergestellt und in verschiedenen Indikationsbereichen als Arzneimittel verwendet: Antiemetica, Neuroleptica, Tranquilizer.

Darüber hinaus gibt es I-Derivate, von denen analgetische, antiinflammatorische, antiallergische, antiasthmatische, antihypertensive, antipsychotische, Dopamin-antagonistische, antidepressive, bronchodilatorische, $\alpha + \beta$-Rezeptoren blockierende, spasmolytische und cardiotonische Wirkungen beschrieben sind. Derivate von I mit R = Hydroxyacyl, worin die Hydroxy-Gruppen aber phenolische sind, werden in Chem.Abstr. 95, 97798p (1981) und 97, 23689y (1982) referiert. Solche 1-Derivate, die gleichzeitig Calcium-antagonistisch wirken, sind bisher nicht bekannt.

Die erfindungsgemäßen Substanzen der allgemeinen Formel (I) zeigen bei sehr guter Verträglichkeit eine starke, lang anhaltende blutdrucksenkende Wirkung; manche wirken gleichzeitig als Calcium-Antagonisten.

Die gemäß Beispiel 1 erhältliche Verbindung senkt den Blutdruck der Ratte nach i.v.-Applikation von 0,1 mg/kg so stark und nachhaltig, wie Prazosin in einer Dosierung von 0,01 mg/kg und ist dabei wesentlich verträglicher als die Vergleichssubstanz.

Das Endprodukt von Beispiel 4 senkt den Blutdruck der Ratte bei gleicher i.v.-Applikation von 1,0 mg/kg nach 3 Stunden noch stärker als Nifedipin oder Verapamil und ist mit einer LD 50 von 127,6 mg/kg Maus i.v. verträglicher als die beiden Vergleichssubstanzen (4,2 mg/kg bzw. 16,8 mg/kg).

Zur Herstellung der erfindungsgemäßen Verbindungen läßt man das 4-(2-Oxo-1-benzimidazolinyl)-piperidin der Formel

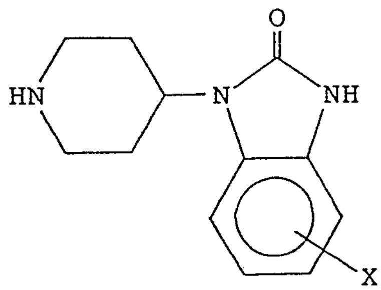

(III),

worin X die oben genannte Bedeutung hat, entweder mit einem Lacton reagieren und setzt gewünschtenfalls das erhaltene 1-(Hydroxyacyl)-Derivat in weiteren Stufen mit Diketen, einem aromatischen Aldehyd und einem 3-Amino-crotonsäureester um oder man läßt das Ausgangsprodukt der Formel III zunächst mit einem Halogenalkanol oder einem Alkylenoxyd reagieren und führt die genannten weiteren Stufen mit dem als Zwischenprodukt erhaltenen 1-(Hydroxyalkyl)-Derivat durch.

Die neuen Substanzen der allgemeinen Formel I können entweder allein oder zusammen mit pharmazeutisch verwendbarem Trägermaterial gegen Bluthochdruck gegeben werden. Die galenische Verarbeitung zu Tabletten, Kapseln, Lösungen, Suspensionen oder Suppositorien erfolgt in der dazu jeweils geeigneten Weise unter Verwendung der dem Fachmann an sich bekannten Hilfsstoffe.

Zur Herabsetzung des Blutdrucks von Hypertonikern wird die Dosierung dem vorgegebenen Überdruck angepaßt und mit relativ geringen Dosen begonnen. Im allgemeinen wird man pro Applikation mit 1 bis 100 mg Wirkstoff auskommen.

Durch die folgenden Beispiele soll die Erfindung näher erläutert, aber nicht auf diese beschränkt werden. Temperaturangaben beziehen sich jeweils auf Celsiusgrade.

Beispiel 1:

50 g 4-(2-Oxo-1-benzimidazolinyl)-piperidin werden in 150 ml ß-Butyrolacton 8 Stunden auf 100° erhitzt. Nach dem Erkalten wird das Reaktionsgemisch mit Äthylacetat verdünnt und über Nacht bei 0° gehalten. Der entstandene Niederschlag wird abgesaugt, mit Äthylacetat ausgekocht und aus Methanol/Äthylacetat umkristallisiert. Das erhaltene 1-(3-Hydroxybutyryl)-4-(2-oxo-1-benzimidazolinyl)-piperidin schmilzt bei 173-175°.

Beispiel 2:

Eine gemäß Beispiel 1 durchgeführte Umsetzung mit γ-Butyrolacton ergibt das entsprechende 1-(4-Hydroxybutyryl)-Derivat mit dem Fp. 148-150°.

Beispiel 3:

Eine gemäß Beispiel 1 durchgeführte Umsetzung mit γ-Valerolacton ergibt das entsprechende 1-(4-Hydroxy-valeroyl)-Derivat mit dem Fp. 120-125°.

Beispiel 4:

Eine Mischung von 4,4 g 1-(3-Acetoacetoxy-propyl)-4-(2-oxo-1-benzimidazolinyl)-piperidin und 1,9 g 3-Nitro-benzaldehyd wird nach Zusatz von 4 Tropfen Piperidin und 3 Tropfen Eisessig in 20 ml Toluol unter einem Wasserabscheider bis zur Beendigung der Wasserbildung erhitzt. Dann dampft man die Mischung am Rotationsverdampfer im Vakuum ein, nimmt den Rückstand in 20 ml Isopropanol auf und erhitzt nach Zusatz von 1,41 g 3-Amino-crotonsäuremethylester unter Rückfluß, bis eine Probe am Dünnschicht-Chromatogramm (Kieselgel 60 F 254, Laufmittel: Chloroform/Methanol = 9 : 1) vollständige Umsetzung anzeigt.

Der Eindampfrückstand wird in Methylenchlorid gelöst und durch Säulen-Chromatographie an Kieselgel 60 gereinigt. Eluiermittel: Aceton, danach Methanol. Die Hauptfraktion kristallisiert nach dem Anreiben mit Isopropyläther. Das erhaltene 2,6-Dimethyl-3-{3-(4-(2-oxo-1-benzimidazolinyl)-1-piperidino]-propyloxy-carbonyl}-4-(3-nitro-phenyl)-5-methoxycarbonyl-1,4-dihydropyridin schmilzt bei 120-125°.

Das verwendete Ausgangsprodukt wird folgendermaßen hergestellt:

Eine Mischung von 50 g 4-(2-Oxo-1-benzimidazolinyl)-piperidin, 20,2 ml 3-Chlor-propanol und 31,75 g $K_2CO_3$ wird in 100 ml Dimethylformamid unter Rühren auf 50 bis 60° erhitzt, bis eine Probe am Dünnschicht-Chromatogramm (Kieselgel 60 F 254, Laufmittel: Äthylacetat/Methanol/2n-$NH_4OH$ = 25 : 8 : 3) kein Ausgangsprodukt mehr erkennen läßt. Der Eindampfrückstand wird mit Wasser behandelt, filtriert, der Niederschlag mit Wasser gewaschen und aus Isopropanol/Aceton umkristallisiert. Fp: 168-170°.

10 g des so erhaltenen Zwischenprodukts behandelt man unter Rühren bei 80° in 50 ml Toluol tropfenweise mit 6,65 ml einer 50 %igen Lösung von Diketen in Aceton und destilliert im Laufe von 3 Stunden 35 ml des Lösungsmittels ab. Das Reaktionsgemisch wird eingedampft und der Rückstand in Äthylacetat gelöst. Auf Zusatz von Isopropyläther kristallisiert das 1-(3-Acetoacetoxypropyl)-4-(2-oxo-1-benzimidazolinyl)-piperidin aus und schmilzt - nach dem Trocknen - bei 112-117°.

Beispiel 5:

45 g des nach Beispiel 1 erhaltenen Produkts werden in 200 ml Xylol unter Rückfluß gerührt und 22,2 g Diketen-Aceton-Addukt langsam zugetropft, wobei Aceton abdestilliert. Nach Beendigung der Reaktion wird abgekühlt, das Xylol von ausgefallenem Öl abdekantiert und letzteres in Chloroform gelöst. Man extrahiert die Chloroformlösung mit 2n NaOH, säuert den alkalischen Extrakt mit 2n HCl an und schüttelt mit Chloroform aus. Diese Chloroformlösung wird nun mit $Na_2SO_4$ sicc. getrocknet, mit Aktivkohle filtriert und das Filtrat eingedampft. Der Rückstand ist ein gelbliches Öl, das bei Dünnschicht-chromatographischer Untersuchung (Kieselgel 60 F 254, Laufmittel: Äthylacetat/Methanol/ 2n-$NH_4OH$ = 25 : 8 : 3) nur einen Fleck zeigt und aus 1-(3-Acetoacetoxy-butyryl)-4-(2-oxo-1-benzimidazolinyl)-piperidin besteht.

7,69 g dieses Zwischenprodukts werden zusammen mit 3 g 3-Nitrobenzaldehyd, 4 Tropfen Piperidin und 3 Tropfen Eisessig in 50 ml Benzol unter einem Wasserabscheider erhitzt, bis die Wasserbildung aufhört. Nach Zusatz von etwas Kieselgel 60 wird das Reaktionsgemisch filtriert, das Filtrat eingedampft und der Rückstand mit 2,29 g 3-Aminocrotonsäuremethylester in 50 ml n-Propanol ca. 8 Stunden unter Rückfluß erhitzt. Der Eindampfrückstand wird in Äthylacetat aufgenommen, die Lösung mit Wasser, 2n HCl und nochmals mit Wasser ausgeschüttelt, mit $Na_2SO_4$ sicc. getrocknet und nach Behandlung mit Aktivkohle filtriert. Der Eindampfrückstand des Filtrats wird aus Isopropanol/Isopropyläther umgefällt und über eine Säule aus Kieselgel 60 chromatographisch gereinigt. Laufmittel: Chloroform/Methanol = 9 : 1. Im gleichen Laufmittel werden die Fraktionen Dünnschicht-chromatographisch auf Kieselgel 60 F 254 überprüft. Das so vorgereinigte Produkt wird aus Isopropanol/Isopropyläther umkristallisiert und schmilzt dann bei 148-153°. Es handelt sich um 4-(2-Oxo-1-benzimidazolinyl)-1-{3-[2,6-dimethyl-4-(3-nitro-phenyl)-5-methoxycarbonyl-1,4-dihydropyridin-3-carbonyloxy]-butyryl}-piperidin.

Beispiel 6:

Entsprechend der Herstellung des zu Beispiel 4 verwendeten Ausgangs- und Zwischenprodukts wurde 4-(2-Oxo-1-benzimidazolinyl)-piperidin mit 6-Chlor-1-hexanol umgesetzt und das dabei erhaltene 1-(6-Hydroxyhexyl)-4-(2-oxo-1-benzimidazolinyl)-piperidin mit Diketen zur Umsetzung gebracht, wodurch das 1-(6-Acetoacetoxy-hexyl)-4-(2-oxo-1-benzimidazolinyl)-piperidin als Öl erhalten wurde.

5,0 g dieses Zwischenprodukts wird mit 2,6 g 2-(N,N-Dimethylcarbamylmethoxy)-benzaldehyd nach Zusatz von 4 Tropfen Piperidin und 3 Tropfen Eisessig in 50 ml Toluol während 4 Stunden unter einem Wasserabscheider zum Rückfluß erhitzt. Den Eindampfrückstand dieser Mischung nimmt man in 50 ml Isopropanol auf und kocht nach Zusatz von 1,43 g 3-Amino-crotonsäuremethylester 16 Stunden unter Rückfluß.

Nach neuerlichem Eindampfen schüttelt man eine Lösung des Rückstandes in Äthylactat mit Wasser aus und danach mit verd. HCl, worauf ein zähes Öl ausfällt. Dieses wird nach Zusatz von verd. NaOH in $CH_2Cl_2$ gelöst und über eine Kieselgel 60-Säule chromatographisch gereinigt. Laufmittel: Methanol. Die Hauptfraktion kristallisiert nach dem Anreiben mit Isopropyläther. Das erhaltene 2,6-Dimethyl-3-{6-[4-(2-oxo-1-benzimidazolinyl)-1-piperidino]-5-hexyloxycarbonyl}-4-(2-N,N-dimethylcarbamylmethoxyphenyl)-5-methoxycarbonyl-1,4-dihydropyridin schmilzt bei 88-95°.

Entsprechend den Beispielen 1 bis 6 werden auch die in folgender Tabelle dargestellten neuen Verbindungen erhalten.

Verbindungen der allgemeinen Formel (I)

(mit R = II und X = H)

| $R_1$ | $R_2$ | $R_3$ | n | m | Fp °C |
|---|---|---|---|---|---|
| 3-$NO_2$ | $CH_3$ | H | 1 | 0 | (amorph) 95-115 |
| 3-$NO_2$ | $CH_3$ | H | 5 | 0 | 85-90 |
| 2-$NO_2$ | $CH_3$ | H | 5 | 0 | 82-90 |
| 2-O.$CH_2$CO.N 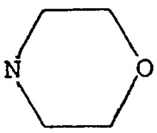 | $CH_3$ | H | 2 | 0 | 90-96 |
| 2-O.$CH_2$CO.N 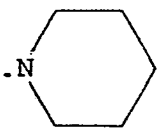 | $CH_3$ | H | 2 | 0 | 83-87 |
| 2-$NO_2$ | $CH_3$ | H | 2 | 0 | 208-213 |
| 2-O.$CH_2CON(C_2H_5)_2$ | $CH_3$ | H | 2 | 0 | 100-115 |
| 2-Cl | $CH_3$ | $CH_3$ | 1 | 1 | 279-280 |
| 2-Cl | $CH_3$ | H | 2 | 0 | (amorph) 105-120 |

**Patentansprüche**

1. Neue Derivate von 4-(2-Oxo-1-benzimidazolinyl)-piperidin der allgemeinen Formel

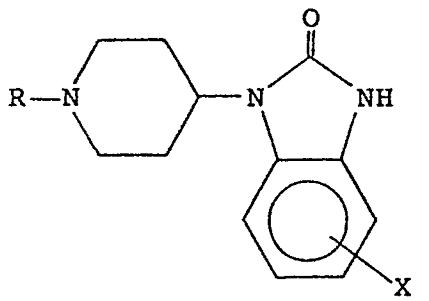

(I),

worin X Wasserstoff oder Halogen und R entweder einen aliphatischen Hydroxyacylrest oder einen Dihydropyridin-3-carbonyloxyalkyl- bzw. einen Dihydropyridin-3-carbonyloxyacyl-Rest der allgemeinen Struktur

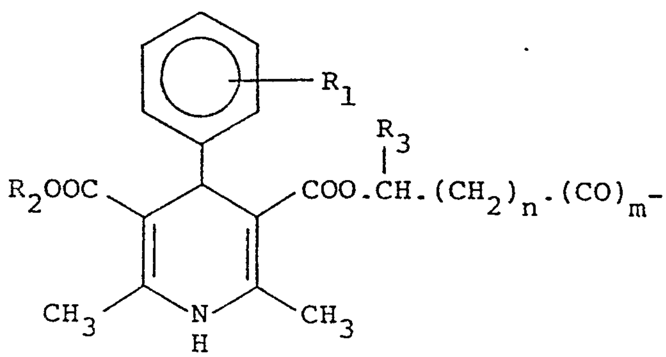

(II)

bedeutet, worin $R_1$ Wasserstoff, Halogen, Nitro, Cyanmethoxy-oder N-subst. Carbamylmethoxy-, $R_2$ ein niederer Alkylrest mit 1 bis 6 C-Atomen, $R_3$ Wasserstoff oder ein niederer Alkylrest, n die Zahl 1 bis 5 und m die Zahl Null oder 1 sein kann.

2. Verfahren zur Herstellung von Verbindungen der allgemeinen Formel (I), dadurch gekennzeichnet, daß man ein 4-(2-Oxo-1-benzimidazolinyl)-piperidin der Formel

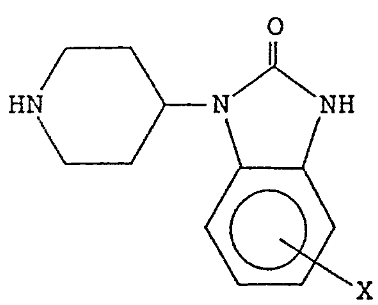

(III),

worin X die oben genannte Bedeutung hat, entweder mit einem Lacton reagieren läßt und gewünschtenfalls das 1-(Hydroxyacyl)-Derivat in weiteren Stufen mit Diketen, einem aromatischen Aldehyd und einem 3-Amino-crotonsäureester umsetzt oder daß man das Ausgangsprodukt der Formel (III) zunächst mit einem Halogenalkanol oder einem Alkylenoxyd zur Reaktion bringt und die genannten weiteren Stufen mit dem als Zwischenprodukt erhaltenen 1-(Hydroxyalkyl)-Derivat durchführt.

3. Verwendung von Verbindungen der allgemeinen Formel (I) mit den oben beschriebenen Substituenten zur Herstellung von blutdrucksenkenden Medikamenten.

Europäisches Patentamt

# EUROPÄISCHER RECHERCHENBERICHT

Nummer der Anmeldung

EP 88 89 0278

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 161 645 (RESEARCH LABORATORIUM DR. C. JANSSEN) --- | | C 07 D 401/04<br>A 61 K 31/415<br>A 61 K 31/445 |
| A | FR-A-1 360 532 (N.V. RESEARCH LABORATORIUM DR. C. JANSSEN) --- | | |
| A | US-A-4 082 755 (JANSSEN PHARMACEUTICA N.V.) --- | | |
| D,A | CHEMICAL ABSTRACTS, Band 95, Nr. 11, 14. September 1981, Seiten 657-658, Zusammenfassung Nr. 97798p, Columbus, Ohio, US; & JP-A-81 39 086 (KYOWA HAKKO KOGYO CO., LTD.) 14-04-1981<br>* Zusammenfassung * --- | | |
| D,A | CHEMICAL ABSTRACTS, Band 97, Nr. 3, 19. Juli 1982, Seite 703, Zusammenfassung Nr. 23689y, Columbus, Ohio, US; H. OBASE et al.: "New antihypertensive agents. II. Studies on new analogs of 4-piperidylbenzimidazolinones", & CHEM. PHARM. BULL. 1982, 30(2), 474-83<br>* Zusammenfassung * --- | | RECHERCHIERTE SACHGEBIETE (Int. Cl.4)<br>C 07 D 401/00<br>A 61 K 31/00 |
| D,A | HELVETICA CHIMICA ACTA, Band XLIII, 4. Juni 1960, Seiten 1298-1313, Basel, CH; A. ROSSI et al.: "Benzimidazol-Derivate und verwandte Heterocyclen V1). Die Kondensation von o-Phenylendiamin mit aliphatischen und alicyclischen beta-Ketoestern"<br>* Seiten 1312-1313 * ----- | | |

Der vorliegende Recherchenbericht wurde für alle Patentansprüche erstellt

| Recherchenort | Abschlußdatum der Recherche | Prüfer |
|---|---|---|
| DEN HAAG | 22-03-1989 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)